# EUROPÄISCHE PATENTSCHRIFT

(11) **EP 2 018 363 B1**
(45) Veröffentlichungstag und Bekanntmachung des Hinweises auf die Patenterteilung: **09.04.2014**
(21) Anmeldenummer: 07728834.8
(22) Anmeldetag: 07.05.2007
(51) Int. Cl.: C07C 209/48, C07C 253/30, C07C 211/11, C07C 255/24, B01J 19/00

(54) **VERFAHREN ZUR HERSTELLUNG VON AMINOALKYLNITRILEN UND DIAMINEN AUS SOLCHEN NITRILEN**
PROCESS FOR PREPARING AMINOALKYLNITRILES AND DIAMINES FROM SUCH NITRILES
PROCEDE DE FABRICATION D'AMINOALKYLNITRILES ET DIAMINES OBTENUES A PARTIR DE TELS NITRILES

(30) Priorität: 09.05.2006 EP 06113718
(43) Veröffentlichungstag der Anmeldung: 28.01.2009
(73) Patentinhaber: BASF SE, 67056 Ludwigshafen (DE)
(72) Erfinder: EBERHARDT, Jan, 68167 Mannheim (DE); HAHN, Thilo, 67292 Kirchheimbolanden (DE); MELDER, Johann-Peter, 67459 Böhl-Iggelheim (DE); FRITZ, Gerhard, 67125 Dannstadt-Schauernheim (DE); MENGER, Volkmar, 67434 Neustadt (DE); HILL, Thomas, 67071 Ludwigshafen (DE)
(86) Internationale Anmeldenummer: PCT/EP2007/054381
(87) Internationale Veröffentlichungsnummer: WO 2007/128803

(56) Entgegenhaltungen:
- EP-A- 1 306 365
- EP-A1- 0 352 504
- EP-A1- 1 221 437
- DD-A- 58 306
- DD-A1- 222 011
- DE-A1- 19 524 971
- US-A- 4 172 091

## Beschreibung

Die vorliegende Erfindung betrifft Verfahren zur Herstellung eines Aminoalkylnitrils durch Umsetzung eines entsprechenden Monoamins mit einem entsprechenden Alkenylnitril sowie Verfahren zur Herstellung eines Diamins aus einem solchen Aminoalkylnitril, sowie Vorrichtungen, welche verwendet werden können, die Verfahren durchzuführen.

Diamine stellen eine wichtige chemische Gruppe dar, die in vielfältigster Weise als Ausgangsstoffe, Zwischen- oder Endprodukte zum Einsatz kommen.

Insbesondere 3-Dimethylaminopropylamin (DMAPA, N,N-Dimethyl-1,3-diaminopropan) stellt ein wichtiges Zwischenprodukt für die technische Produktion beispielsweise von Flüssigseifen dar. DMAPA dient zudem als Ausgangsprodukt für die Herstellung von Koagulationsmitteln und soll selbst antikorrosive Eigenschaften aufweisen.

Häufig werden Diamine ebenso wie deren Amin-Analoga durch Reduktion von Nitrilen hergestellt. Diese Reaktion ist insbesondere dann von Vorteil, wenn primäre Amine erhalten werden sollen.

Die entsprechenden Nitrile, welche bereits eine Aminofunktion aufweisen können, werden beispielsweise aus Alkenylnitrilen durch Addition eines Monoamins an die C-C-Doppelbindung erhalten.

Es stellt einen besonderen Vorteil dar, wenn zur Herstellung von Diaminen aus Nitrilen ein integrales Herstellverfahren bzw. eine integrale Vorrichtung zum Einsatz kommen kann. Hierbei wird der zunächst erhaltene Produktstrom, welcher das Aminonitril aufweist, direkt oder nach Aufreinigung eingesetzt, um in einem weiteren Schritt zum Diamin umgesetzt zu werden.

Hierbei ist für die Reduktionsreaktion des Nitrils die Qualität des Aminoalkylnitril-Produktstromes aus der ersten Umsetzung (Monoamin und Alkenylnitril) von entscheidender Bedeutung, insbesondere für den Verbrauch des bei der Reduktion eingesetzten Katalysators.

Im Falle des 3-Dimethylaminopropylamins ist daher die Herstellung von 3-Dimethylaminopropionitril (DMAPN) von Bedeutung, wobei letzteres aus Acrylnitril und Dimethylamin hergestellt wird. Es sind in der Literatur integrale Verfahren, insbesondere zur Herstellung von DMAPA, beschrieben, die also sowohl den Additions- als auch den Reduktionsschritt umfassen. Darüber hinaus sind jeweils für die einzelnen Stufen optimierte Verfahren beschrieben.

Diese Verfahren können zunächst in solche Verfahren unterteilt werden, die einerseits batchweise oder zumindest semi-batchweise durchgeführt werden und Verfahren, die in kontinuierlicher Fahrweise durchgeführt werden können.

Ein integrales Herstellverfahren, durch welches beispielsweise DMAPA batchweise hergestellt werden kann, ist in der deutschen Patentanmeldung mit der Anmelde-Nr. 10 2005 052 457.5, welche auf die Anmelderin zurückgeht, beschrieben.

Es hat sich jedoch als günstig erwiesen, alternativ zu solchen zumindest teilweise als Batch betriebenen Verfahren kontinuierliche Verfahren bereitzustellen. Hierbei ist es insbesondere wichtig, für den ersten Schritt ein für den zweiten (Reduktions-)Schritt optimiertes Verfahren bereitzustellen, um so im zweiten (Reduktions-)Schritt ebenfalls eine optimierte Diaminproduktion zu erhalten.

Eine hohe Optimierung bedeutet in diesem Zusammenhang insbesondere, dass eine hohe Einzel- sowie Gesamtausbeute an Diamin erzielt wird. Darüber hinaus ist es wichtig, dass die Ausbeute mit einer möglichst hohen Einzel- sowie Gesamtumsätze erfolgt. Schließlich ist weiterhin ein Qualitätskriterium darin zu sehen, mit welchen Raum/Zeit-Ausbeuten ein solches Verfahren durchgeführt werden kann. Letztendlich spielt auch der Verbrauch des bei der Reduktion eingesetzten Katalysators eine wichtige Rolle.

Ein Verfahren zur Herstellung von 3-Dimethylaminopropionitril ist beispielsweise in DD-A 58 306 beschrieben. Darin wird vorgeschlagen, dass vorgebildetes 3-Dimethylaminopropionitril als Lösungs- und Verdünnungsmittel bei der Umsetzung von Acrylsäurenitril mit Dimethylamin eingesetzt werden soll, um aufgrund der exothermen Reaktion und des Siedeverhaltens des Dimethylamins eine erforderliche verbesserte Wärmeverteilung und -abfuhr zu erreichen. Dies soll die Ausbeute an 3-Dimethylaminopropionitril erhöhen.

DD-A 222 011 beschreibt ein Verfahren zur kontinuierlichen Herstellung von DMAPN, wobei hier die Reaktionskomponenten in flüssiger Phase in einem Rohrreaktor zunächst in einer Mischkammer vorgelegt und dann stufenweise vermischt und über die gesamte Reaktorlänge stufenweise zur Reaktion gebracht werden.

Schließlich beschreibt DE-A 27 09 966 die Herstellung von DMAPN, wobei jedoch das Dimethylamin gasförmig eingesetzt wird und die Umsetzung in einem Blasensäulenreaktor derart durchgeführt wird, dass die Reaktionspartner im Gegenstrom geführt werden.

Obwohl solche optimierten Verfahren im Stand der Technik bekannt sind, besteht nach wie vor ein Bedarf, weitere Verfahren und entsprechende Vorrichtungen bereitzustellen, um die Herstellung eines Aminoalkylnitrils und/oder die daraus resultierende Herstellung eines Diamins zu optimieren.

Eine Aufgabe der vorliegenden Erfindung liegt somit darin, Verfahren und Vorrichtungen bereitzustellen, die eine solche Optimierung erlauben.

Die Aufgabe wird gelöst durch ein Verfahren zur Herstellung eines Aminoalkylnitrils durch Umsetzung eines entsprechenden Monoamins mit einem entsprechenden Alkenylnitril in kontinuierlicher Fahrweise, die Schritte enthaltend
(a) Zuführen des entsprechenden Monoamins in einen kontinuierlich gefahrenen Reaktionsstrom;
(b) Zuführen des entsprechenden Alkenylnitrils in den Reaktionsstrom, wobei dieser bei Zugabe bereits das Aminoalkylnitril enthält;
(c) Umsetzung des Reaktionsstromes in einem ersten Reaktionsbereich; und
(d) zumindest teilweises Überführen des Reaktionsstromes in zumindest einen zweiten Reaktionsbereich zur weiteren Umsetzung.

Es hat sich nämlich gezeigt, dass insbesondere bei der kontinuierlichen Fahrweise es vorteilhaft ist, das Alkenylnitril nicht zu reinem Monoamin zuzugeben und darüber hinaus die Reaktionsbereiche derart zu trennen, dass die Reaktion zumindest zweistufig durchgeführt werden kann.

Die Durchführung des oben beschriebenen optimierten Verfahrens hat weiterhin den Vorteil, dass ein Aminoalkylnitrilstrom erhalten werden kann, der besonders geeignet ist, bei einer nachfolgenden Reduktion des Nitrils zum entsprechenden Diamin eingesetzt zu werden.

Dem erfindungsgemäßen Verfahren zur Herstellung eines Aminoalkylnitrils liegt somit die Additionsreaktion zugrunde, bei der die Anlagerung eines primären oder sekundären Monoamins an die C-C-Doppelbindung eines Alkenylnitrils erfolgt.

Im Rahmen der vorliegenden Erfindung bedeuten die Begriffe "entsprechendes Alkenylnitril" sowie "entsprechendes Monoamin", dass diese derart ausgewählt werden, dass nach dem oben beschriebenen Syntheseschritt das Aminoalkylnitril mit der gewünschten Strukturformel erhalten wird.

Handelt es sich beispielsweise bei dem Alkenylnitril um Acrylsäurenitril (ACN) und bei dem Monoamin um Dimethylamin (DMA), so erfolgt eine Umsetzung der Edukte zum gewünschten 3-Dimethylaminopropionitril (DMAPN). Anders ausgedrückt ist für den Fall, dass in dem erfindungsgemäßen Verfahren DMAPN erhalten werden soll, klar ersichtlich, dass das entsprechende Alkenylnitril ACN und das entsprechende Monoamin DMA sind.

Das Alkenylnitril ist vorzugsweise ein C₂-C₄-Alken, das geradekettig oder verzweigt sein kann, bei dem ein Wasserstoffatom durch die Cyanogruppe ausgetauscht ist.

Der Begriff C₂-C₄-Alken bedeutet ein Alken mit 2 bis 4 C-Atomen, das mindestens eine C-C-Doppelbindung enthält. Vorzugsweise ist genau eine C-C-Doppelbindung in α,β-Position zur Cyanogruppe vorhanden. Beispiele für C₂-C₄-Alkene sind Ethen, Propen, 1-Buten, 2-Buten, 2-Methylpropen.

Beispiele für Alkenylnitrile sind Acrylnitril, But-2-ensäurenitril, Methacrylsäurenitril, Pent-2-ensäurenitril, 2-Ethylacrylsäurenitril, 2-Methylbut-2-ensäurenitril sowie 3-Methylbut-2-ensäurenitril.

Bevorzugt ist ACN.

Bei dem Monoamin handelt es sich bevorzugt um ein primäres oder sekundäres Amin der allgemeinen Formel R¹R²NH, bei dem R¹, R² unabhängig voneinander H oder C₁-C₄-Alkyl mit der Maßgabe sind, dass wenigstens ein Rest R¹, R² nicht Wasserstoff ist.

C₁-C₄-Alkyl bedeutet Methyl, Ethyl, n-Propyl, i-Propyl, 1-n-Butyl, 2-n-Butyl, i-Butyl, t-Butyl.

Bevorzugt ist DMA.

In einer besonders bevorzugten Ausführungsform handelt es sich bei dem Aminoalkylnitril um 3-Dimethylaminopropionitril.

Das erfindungsgemäße Verfahren zur Herstellung des Aminoalkylnitrils soll in kontinuierlicher Fahrweise erfolgen. Dies ermöglicht einen besonders effizienten Umsatz verbunden mit einer hohen Raum/Zeit-Ausbeute.

Demzufolge erfolgt in den Schritten (a) und (b) des erfindungsgemäßen Verfahrens zur Herstellung eines Aminoalkylnitrils die Zuführung des entsprechenden Monoamins bzw. die Zuführung des entsprechenden Alkenylnitrils in einen kontinuierlich gefahrenen Reaktionsstrom. Hierbei ist zu beachten, dass das entsprechende Alkenylnitril in den Reaktionsstrom derart zugeführt wird, dass dieser bei Zugabe bereits das Aminoalkylnitril enthält. Hierdurch wird gewährleistet, dass das im Reaktionsstrom befindliche Monoamin gegebenenfalls in ausreichender Verdünnung vorliegt, um die bei Zugabe des Alkenylnitrils entstehende Reaktionswärme entsprechend kontrollieren zu können. Dies kann weiterhin dadurch erreicht werden, dass nach Zuführung des entsprechenden Alkenylnitrils der Reaktionsstrom einen Wärmetauscher durchläuft, um gegebenenfalls Wärme abführen zu können. Die eigentliche Umsetzung des Monoamins mit dem Alkenylnitril erfolgt in einem ersten Reaktionsbereich. Dieser erste Reaktionsbereich kann eine Zone in einem Reaktor oder einen Reaktor als solchen darstellen.

Typischerweise unterscheidet sich ein Reaktionsbereich von Leitungsbereichen, die lediglich der Zu- beziehungsweise Abfuhr des Reaktionsstromes dienen, durch die Verweildauer des Stromes in den entsprechenden Bereichen. Vorzugsweise beträgt die mittlere Verweilzeit in einem Reaktionsbereich mehr als 15 min., mehr bevorzugt mehr als 30 min., wobei die mittlere Verweilzeit für Leitungsbereiche in der Regel unter 10 min. liegt.

Weiterhin erfolgt im erfindungsgemäßen Verfahren zur Herstellung eines Aminoalkylnitrils eine zumindest teilweise Überführung des Reaktionsstromes in zumindest einen zweiten Reaktionsbereich, der vom ersten Reaktionsbereich räumlich getrennt ist, zur weiteren Umsetzung. Der zweite Reaktionsbereich kann eine weitere Zone in dem oben erwähnten Reaktor oder ein weiterer Reaktor bzw. eine Reaktorkaskade sein. Die räumliche Trennung von erstem und zweitem Reaktionsbereich kann beispielsweise durch Rohrleitungen erfolgen.

Es ist vorteilhaft, wenn nicht der gesamte Reaktionsstrom in zumindest einen zweiten Reaktionsbereich überführt wird, sondern ein Teil des Reaktionsstromes, welcher bereits gebildetes Aminoalkylnitril enthält, in einer Kreisstromfahrweise derart rückgeführt wird, dass das entsprechende Monoamin und das entsprechende Alkenylnitril zumindest nach Beginn der Aminoalkylnitrilbildung im ersten Reaktionsbereich jeweils in den das Aminoalkylnitril enthaltenen Reaktionsstrom zugeführt werden können.

Daher besteht eine bevorzugte Ausführungsform des erfindungsgemäßen Verfahrens zur Herstellung eines Aminoalkylnitrils darin, dass zumindest die Schritte (a) bis (c) des erfindungsgemäßen Verfahrens zur Herstellung eines Aminoalkylnitrils in Kreisstromfahrweise durchgeführt werden.

Es hat sich weiterhin als vorteilhaft erwiesen, wenn das entsprechende Monoamin und das entsprechende Alkenylnitril in flüssiger Form zugeführt werden. Dies erlaubt einen höheren Umsatz bei besserer Raum/Zeit-Ausbeute im Vergleich zu Verfahren, bei denen das Monoamin in gasförmigem Zustand dem Reaktionsgemisch zugeführt wird. Daher weist die Zuführung des entsprechenden Alkenylnitrils sowie des entsprechenden Monoamins eine geeignete Temperatur sowie einen geeigneten Druck auf, die gewährleisten, dass diese Edukte zur Herstellung des Aminoalkylnitrils in flüssiger Form vorliegen.

Daher ist ein bevorzugter Gegenstand des erfindungsgemäßen Verfahrens zur Herstellung eines Aminoalkylnitrils die Zuführung des entsprechende Alkenylnitrils sowie des entsprechenden Monoamins in flüssiger Form.

Weiterhin ist bevorzugt, dass bei der Zugabe des entsprechenden Alkenylnitrils der Reaktionsstrom einen Mindestüberschuss an entsprechendem Monoamin von 0,1 Mol-% in Bezug auf das Alkenylnitril aufweist. Weiterhin bevorzugt weist der molare Überschuss an Monoamin in Bezug auf das Alkenylnitril einen Wert auf, der im Bereich von 0,1 bis 20 % liegt. Weiterhin bevorzugt liegt der Wert im Bereich von 1 bis 10 Mol-%. Insbesondere liegt der Wert im Bereich von 2 bis 8 Mol-%.

Der Überschuss an Monoamin in Bezug auf das Alkenylnitril kann durch Zugabe der beiden Edukte im entsprechenden Verhältnis erreicht werden, sofern in Richtung des Reaktionsstromes das Zuführen des Monoamins räumlich vor der Zuführung des Alkenylnitrils erfolgt.

Die Umsetzung des Reaktionsstromes bzw. des Monoamins mit dem Alkenylnitril im Reaktionsstrom erfolgt in zwei unterschiedlichen Reaktionsbereichen. Dies hat den Vorteil, dass für die Umsetzung unterschiedliche Reaktionsbedingungen gewählt werden können. Hierdurch kann eine vollständigere Umsetzung des Alkenylnitrils bei gleichzeitiger Verminderung des Anteils an Nebenprodukten im Reaktionsstrom erreicht werden.

Vorzugsweise erfolgt die Umsetzung in Schritt (c) des erfindungsgemäßen Verfahrens zur Herstellung eines Aminoalkylnitrils derart, dass der Verbrauch an Alkenylnitril im ersten Reaktionsbereich 80 bis 98 % beträgt. Dies bedeutet, dass bei Eintritt des Reaktionsstromes in den ersten Reaktionsbereich der Strom einen ersten Konzentrationswert an Alkenylnitril und nach Verlassen des ersten Reaktionsbereichs eine zweite Konzentration aufweist, wobei der von 1 substrahierte Quotient aus zweiter Konzentration zu erster Konzentration einen Wert von 0,8 bis 0,98 aufweist.

Weiterhin bevorzugt liegt der Umsatz im ersten Reaktionsbereich im Bereich von 90 bis 95%.

Weiterhin ist bevorzugt, dass nach der Umsetzung in Schritt (d) des erfindungsgemäßen Verfahrens zur Herstellung eines Aminoalkylnitrils der Restgehalt an Alkenylnitril im Reaktionsstrom unter 1 Gew.-% liegt.

Darüber hinaus können weitere Reaktionsbereiche vorhanden sein. Für diesen Fall ist es ausreichend, wenn der in Richtung des Reaktionsstromes zuletzt vorhandene Reaktionsbereich derart ausgestaltet ist, dass der oben angegebene Restgehalt an Alkenylnitril im Reaktionsstrom unter 1 Gew.-% liegt.

Bei Vorhandensein von mehr als zwei Reaktionsbereichen kann gegebenenfalls die Umsetzung im in Richtung des Reaktionsstromes ersten Reaktionsbereich niedriger als oben angegeben sein. Wie oben bereits ausgeführt wurde, liegt ein Vorteil im erfindungsgemäßen Verfahren zur Herstellung eines Aminoalkylnitrils darin, dass die Umsetzung zum Alkenylnitril in zwei unterschiedlichen Reaktionsbereichen durchgeführt wird. Hierdurch können für die Umsetzung unterschiedliche Reaktionsbedingungen gewählt werden. Es hat sich dabei als vorteilhaft erwiesen, dass der Reaktionsstrom im ersten und im zweiten Reaktionsbereich bei der Umsetzung unterschiedliche Temperaturen aufweist. Insbesondere ist bevorzugt, wenn der Reaktionsstrom im ersten Reaktionsbereich bei der Umsetzung eine höhere Temperatur als im zumindest zweiten Reaktionsbereich aufweist.

Vorzugsweise liegt die Temperatur des Reaktionsstromes im ersten Reaktionsbereich im Bereich von 20°C bis 110°C. Mehr bevorzugt beträgt der Bereich 60°C bis 80°C.

Die Temperatur des Reaktionsstromes im zumindest zweiten Reaktionsbereich liegt vorzugsweise im Bereich von 0 bis 100°C, insbesondere im Bereich von 30 bis 60°C. Sofern zumindest noch ein weiterer Reaktionsbereich vorhanden ist, kann die im Vergleich zum ersten Reaktionsbereich unterschiedliche, vorzugsweise niedrigere Temperatur auch erst in dem zumindest weiteren Reaktionsbereich vorliegen.

Darüber hinaus kann der Druck in dem ersten und zweiten Reaktionsbereich unterschiedlich sein. Ebenso kann jedoch der Druck auch gleich sein, was bevorzugt ist. Vorzugsweise liegt in dem ersten und zweiten Reaktionsbereich ein Überdruck im Bereich von 1 bar bis 20 bar vor. Es ist daher bevorzugt im erfindungsgemäßen Verfahren zur Herstellung eines Aminoalkylnitrils, dass der Überdruck in dem ersten und zweiten Reaktionsbereich gleich oder verschieden ist und im Bereich von 1 bar bis 20 bar liegt. Vorzugsweise beträgt der Überdruck 2 bis 5 bar. Weiterhin ist bevorzugt, dass die Zuführungen für das entsprechende Monoamin sowie das entsprechende Alkenylnitril einen ebenso hohen Überdruck aufweisen.

Es ist bekannt, dass bei der Addition eines Monoamins an die C-C-Doppelbindung eines Alkenylnitrils zugegebenes Wasser die Reaktion katalysiert. Es ist daher weiterhin bevorzugt, dass dem Reaktionsstrom vor der Umsetzung im ersten Reaktionsbereich Wasser zugeführt wird, bevor der Reaktionsstrom den ersten Reaktionsbereich erreicht.

Das Wasser kann beispielsweise in Form einer zusätzlichen Zuführung in den Reaktionsstrom gelangen. Weiterhin ist es möglich, dass zumindest teilweise das Monoamin in Form einer wässrigen Lösung dem Reaktionsstrom zugeführt wird. Dies kann beispielsweise in Form einer 30 bis 70 gew.-%igen wässrigen Monoaminlösung erfolgen. Zudem kann das eingesetzte Alkenylnitril Wasser enthalten.

Vorzugsweise enthält das erfindungsgemäße Verfahren zur Herstellung eines Aminoalkylnitrils einen weiteren Schritt (e), bei dem der Reaktionsstrom nach der Umsetzung aus dem zweiten Reaktionsbereich ausgeführt wird.

Weiterhin kann dem Reaktionsstrom nach der Umsetzung im zweiten Reaktionsbereich nicht umgesetztes Monoamin im zweiten Reaktionsbereich oder außerhalb des zweiten Reaktionsbereichs entzogen werden. Dies kann beispielsweise destillativ erfolgen. Hierzu kann ein weiterer Behälter, wie beispielsweise eine Destillationskolonne, eingesetzt werden. Es ist bevorzugt, dass ein solcher Entzug des nicht umgesetzten Monoamins außerhalb des zweiten Reaktionsbereiches, insbesondere diesem nachgeschaltet, durchgeführt wird. Das so zurückgewonnene Monoamin kann dem Reaktionsstrom, bevor dieser den ersten Reaktionsbereich erreicht, wieder zugeführt werden. Dies kann vollständig oder teilweise erfolgen. Es ist ebenfalls denkbar, dass das zurückgewonnene nicht umgesetzte Monoamin weiter verarbeitet oder entsorgt wird.

Ein weiterer Gegenstand der vorliegenden Erfindung ist ein Verfahren zur Herstellung eines Diamins aus einem entsprechenden Aminoalkylnitril die Schritte enthaltend:
(a) Einleiten des in Schritt (e) des erfindungsgemäßen Verfahrens zur Herstellung eines Aminoalkylnitrils ausgeleiteten Reaktionsstromes in einen Reduktionsbereich; und
(b) Reduktion des im Reaktionsstrom befindlichen Aminoalkylnitrils zum entsprechenden Diamin.

Basierend auf dem Reaktionsstrom, wie er sich nach zumindest zweistufiger Umsetzung aus dem erfindungsgemäßen Verfahren zur Herstellung eines Aminoalkylnitrils ergibt, besteht ein weiterer Gegenstand der vorliegenden Erfindung in der Reduktion des entsprechenden Aminoalkylnitrils zum Diamin. Hierbei wird somit in einem zweiten Reaktionsschritt die Nitrilgruppe in die Oxidationsstufe eines Amins überführt. Das so erhaltene Produkt enthält neben dieser Aminofunktion eine weitere Aminofunktion, die durch das Monoamin eingeführt wurde.

In einer bevorzugten Ausführungsform erfolgt die Reduktion mit Hilfe von Wasserstoff. Dieser kann als solcher oder als Teil eines Gasgemisches eingesetzt werden. Somit wird die Nitrilgruppe des Aminoalkylnitrils in eine primäre Aminfunktion umgewandelt.

Vor Schritt (b) des erfindungsgemäßen Verfahrens zur Herstellung eines Diamins kann dem Reaktionsstrom Ammoniak zugeführt werden.

Vorzugsweise werden die Schritte (a) und (b) des erfindungsgemäßen Verfahrens zur Herstellung eines Diamins in kontinuierlicher Fahrweise durchgeführt.

Insbesondere ist bevorzugt, dass nach der Reduktion in Schritt (b) das gebildete Rohprodukt zumindest teilweise derart zurückgeführt wird, dass ein Kreisstrom entsteht.

Bei Vorliegen eines solchen Kreisstroms ist es bevorzugt, dass für den Fall, dass Wasserstoff und Ammoniak eingesetzt werden, diese dem Kreisstrom zugeführt werden, bevor der aus der ersten Stufe ausgeführte Reaktionsstrom in den Kreisstrom eingeleitet wird.

Dabei ist es ganz besonders bevorzugt, wenn der Kreisstrom nach Einleiten von Ammoniak sowie Wasserstoff und vor Einleiten des aus der ersten Stufe ausgeleiteten Reaktionsstromes der Kreisstrom einen Wärmetauscher durchläuft, der den Kreisstrom erwärmt.

Das Molverhältnis von Ammoniak zu Aminoalkylnitril beträgt beispielsweise etwa 5. Für den Fall, dass Wasserstoff zur Hydrierung eingesetzt wird, kann ein Gesamtdruck im Bereich von 50 bis 300 bar eingestellt werden. Der Gesamtdruck ergibt sich aus der Summe der Partialdrücke aus Ammoniak, Aminoalkylnitril, Wasserstoff sowie gegebenenfalls nicht umgesetzter Edukte und gebildeter Produkte sowie gegebenenfalls Wasser bei der entsprechenden Temperatur und wird zweckmäßigerweise durch Aufpressen von Wasserstoff auf den gewünschten Wert eingestellt. In einer bevorzugten Ausführungsform werden die Schritte (a) und (b) des erfindungsgemäßen Verfahrens zur Herstellung des Diamins in kontinuierlicher Fahrweise durchgeführt.

Zweckmäßigerweise wird insbesondere bei der Verwendung von Wasserstoff ein Kata-lysator eingesetzt. Die Reduktion findet vorzugsweise an einem Katalysator-Festbett statt. Hierbei ist eine Sumpf- sowie Rieselfahrweise möglich. Geeignete Katalysatoren zur Hydrierung des Aminoalkylnitrils zum entsprechenden Diamin sind in EP-B 742 045 oder EP-B 892 77 beschrieben. Die maximale Temperatur im Reduktionsbereich während der Reduktion beträgt vorzugsweise 150°C.

Als Katalysatoren zur Hydrierung der Nitril-Funktion zum Amin können insbesondere Katalysatoren eingesetzt werden, die als aktive Spezies ein oder mehrere Elemente der 8. Nebengruppe des Periodensystems (Fe, Co, Ni, Ru, Rh, Pd, Os, Ir, Pt), bevorzugt Fe, Co, Ni, Ru oder Rh, besonders bevorzugt Co oder Ni enthalten. Darin eingeschlossen sind sogenannte Skelett-Katalysatoren (auch als Raney^{®}-Typ bezeichnet, nachfolgend auch: Raney-Katalysator), die durch Auslaugen (Aktivierung) einer Legierung aus hydrieraktivem Metall und einer weiteren Komponente (bevorzugt Al) erhalten werden. Die Katalysatoren können zusätzlich einen oder mehrere Promotoren enthalten.

Die Katalysatoren können als Vollkatalysatoren oder geträgert eingesetzt werden. Als Träger kommen bevorzugt Metalloxide wie Al₂O₃, SiO₂, ZrO₂, TiO₂, Gemische von Metalloxiden oder Kohlenstoff (Aktivkohlen, Ruß, Graphit) zur Anwendung.

Die oxidischen Katalysatoren werden vor dem Einsatz außerhalb des Reaktors oder im Reaktor durch Reduktion der aktiven Metalloxide in einem Wasserstoff enthaltenden Gasstrom bei erhöhter Temperatur aktiviert. Wenn die Katalysatoren außerhalb des Reaktors reduziert werden, kann danach eine Passivierung durch einen Sauerstoff enthaltenden Gasstrom oder die Einbettung in ein inertes Material erfolgen, um eine unkontrollierte Oxidation an Luft zu vermeiden und einen sicheren Umgang zu ermöglichen.

Besonders bevorzugte Festbett-Katalysatoren sind die in EP-A 1 742 045 offenbarten Cobalt-Vollkontakte, dotiert mit Mn, P und Alkalimetall (Li, Na, K, Rb, Cs). Die katalytisch aktive Masse dieser Katalysatoren besteht vor der Reduktion mit Wasserstoff aus 55 bis 98 Gew.-%, insbesondere 75 bis 95 Gew.-%, Cobalt, 0,2 bis 15 Gew.-% Phosphor, 0,2 bis 15 Gew.-% Mangan und 0,05 bis 5 Gew.-% Alkalimetall, insbesondere Natrium, jeweils berechnet als Oxid.

Weitere geeignete Katalysatoren sind die in EP-A 963 975 offenbarten Katalysatoren, die vor der Behandlung mit Wasserstoff 22 bis 40 Gew.-% ZrO₂, 1 bis 30 Gew.-% sauerstoffhaltige Verbindungen des Kupfers, berechnet als CuO, 15 bis 50 Gew.-% sauerstoffhaltige Verbindungen des Nickels, berechnet als NiO, wobei das molare Ni : Cu-Verhältnis größer 1 ist, 15 bis 50 Gew.-% sauerstoffhaltige Verbindungen des Cobalts, berechnet als CoO, 0 bis 10 Gew.-% sauerstoffhaltige Verbindungen des Aluminiums und/oder Mangans, berechnet als Al₂O₃ beziehungsweise MnO₂, und keine sauerstoffhaltigen Verbindungen des Molybdäns enthalten; beispielsweise der in diesem Dokument offenbarte Katalysator A mit der Zusammensetzung 33 Gew.-% Zr, berechnet als ZrO₂, 28 Gew.-% Ni, berechnet als NiO, 11 Gew.-% Cu, berechnet als CuO und 28 Gew.-% Co, berechnet als CoO.

Weiterhin geeignet sind die in EP-A 696 572 offenbarten Katalysatoren, die vor der Reduktion mit Wasserstoff 20 bis 85 Gew.-% ZrO₂, 1 bis 30 Gew.-% sauerstoffhaltige Verbindungen des Kupfers, berechnet als CuO, 30 bis 70 Gew.-% sauerstoffhaltige Verbindungen des Nickels, berechnet als NiO, 0,1 bis 5 Gew.-% sauerstoffhaltige Verbindungen des Molybdäns, berechnet als MoO₃, und 0 bis 10 Gew.-% sauerstoffhaltige Verbindungen des Aluminiums und/oder Mangans, berechnet als Al₂O₃ beziehungsweise MnO₂ enthalten. Beispielsweise sei der in dieser Schrift konkret offenbarte Katalysator mit der Zusammensetzung 31,5 Gew.-% ZrO₂, 50 Gew.-% NiO, 17 Gew.-% CuO und 1,5 Gew.-% MoO₃ erwähnt. Ebenso geeignet sind die in WO-A 99/44984 beschriebenen Katalysatoren enthaltend (a) Eisen oder eine Verbindung auf der Basis von Eisen oder deren Gemische, (b) von 0,001 bis 0,3 Gew.-% bezogen auf (a) eines Promotors auf der Basis von 2, 3, 4 oder 5 Elementen ausgewählt aus der Gruppe Al, Si, Zr, Ti, V, (c) von 0 bis 0,3 Gew.-% bezogen auf (a) einer Verbindung auf der Basis eines Alkali- und/oder Erdalkalimetalls, sowie (d) von 0,001 bis 1 Gew.-% bezogen auf (a) Mangan.

Die Reduktion kann bei geeigneter Reaktionsführung adiabatisch erfolgen.

Der aus der Reduktion erhaltene Strom enthält neben dem Produkt auch Ammoniak und Wasserstoff, die gegebenenfalls nach einer Abtrennung wieder zurückgeführt werden können.

Ein weiterer Gegenstand ist eine Vorrichtung zur Herstellung eines Aminoalkylnitrils durch Umsetzung eines entsprechenden Monoamins mit einem entsprechenden Alkenylnitril in kontinuierlicher Fahrweise enthaltend
- einen ersten Reaktionsbereich enthaltend
- einen Einlass mit einer Zuleitung, wobei die Zuleitung
- einen ersten Einlass, der geeignet ist, der Zuleitung das Monoamin zuzuführen;
- einen zweiten Einlass, der geeignet ist, das Alkenylnitril der Zuleitung zuzuführen und der zwischen erstem Einlass und Einlass des ersten Reaktionsbereiches liegt, aufweist;
- einen Auslass, der mit der Zuleitung verbunden ist; und
- eine Überführung zu einem zweiten Reaktionsbereich; und
- einen zweiten Reaktionsbereich,
wobei die Zuleitung das Aminoalkylnitril enthält.

Die oben beschriebene Vorrichtung zur Herstellung eines Aminoalkylnitrils ist eine bevorzugte Vorrichtung, um das erfindungsgemäße Verfahren zur Herstellung eines Aminoalkylnitrils durchzuführen.

Die Vorrichtung enthält einen ersten Reaktionsbereich, welcher einen Einlass mit einer Zuleitung enthält, wobei die Zuleitung weiterhin einen ersten Einlass, der geeignet ist, der Zuleitung das Monoamin zuzuführen sowie einen zweiten Einlass, der geeignet ist, das Alkenylnitril der Zuleitung zuzuführen, aufweist. Hierbei befindet sich der zweite Einlass zwischen erstem Einlass und Einlass des ersten Reaktionsbereiches. Weiterhin weist der erste Reaktionsbereich einen Auslass auf, der mit der Zuleitung verbunden ist. Hierdurch kann das erfindungsgemäße Verfahren zur Herstellung eines Aminoalkylnitrils in kontinuierlicher Kreisfahrweise durchgeführt werden bzw. die erfindungsgemäße Vorrichtung zur Herstellung eines Aminoalkylnitrils betrieben werden. Schließlich weist der erste Reaktionsbereich eine Überführung auf, die zu einem zweiten Reaktionsbereich führt. Demzufolge enthält die erfindungsgemäße Vorrichtung zur Herstellung eines Aminoalkylnitrils ebenfalls einen zweiten Reaktionsbereich. Die erfindungsgemäße Vorrichtung zur Herstellung eines Aminoalkylnitrils enthält hierbei das Aminoalkylnitril in der Zuleitung.

Letzteres gewährleistet, dass bei Zuleitung des Alkenylnitrils über den zweiten Einlass eine Verdünnung durch das Aminoalkylnitril erfolgt und somit keine Zuleitung des Alkenylnitrils zu reinem Monoamin erfolgt.

Der erste und zweite Reaktionsbereich der erfindungsgemäßen Vorrichtung zur Herstellung eines Aminoalkylnitrils können in einem Reaktorbehälter in Form von unterschiedlichen Zonen vorliegen. Es ist jedoch bevorzugt, dass der erste und zweite Reaktionsbereich unterschiedliche Reaktorbehälter bzw. eine Behälterkaskade darstellen.

Diese beiden Reaktorbehälter können durch eine Rohrleitung miteinander verbunden sein.

Darüber hinaus ist ein weiterer Gegenstand der vorliegenden Erfindung eine Vorrichtung zur Herstellung eines Diamins aus einem entsprechenden Aminoalkylnitril enthaltend
- eine Vorrichtung zur Herstellung eines Aminoalkylnitrils wie oben beschrieben;
   und
- einen Reduktionsbereich.

Bei dem Reduktionsbereich handelt es sich vorzugsweise um einen Festbettreaktor.

Wie bereits oben ausgeführt wurde, ist die erfindungsgemäße Vorrichtung zur Herstellung eines Aminoalkylnitrils eine bevorzugte Vorrichtung zur Durchführung eines erfindungsgemäßen Verfahrens zur Herstellung eines Aminoalkylnitrils.

Daher ist ein weiterer Gegenstand der vorliegenden Erfindung die Verwendung einer erfindungsgemäßen Vorrichtung zur Herstellung eines Aminoalkylnitrils durch Umsetzung eines entsprechenden Monoamins mit einem entsprechenden Alkenylnitril in kontinuierlicher Fahrweise.

Darüber hinaus ist die erfindungsgemäße Vorrichtung zur Herstellung eines Diamins geeignet, das erfindungsgemäße Verfahren zur Herstellung eines Diamins durchzuführen.

Demzufolge ist ein weiterer Gegenstand die Verwendung einer erfindungsgemäßen Vorrichtung zur Herstellung eines Diamins aus einem entsprechenden Aminoalkylnitril.

Die Erfindung wird anhand der nachfolgenden Figuren und Beispiele näher erläutert.

Es zeigen
- Figur 1: das schematische Fließbild für ein beispielhaftes Verfahren zur Herstellung eines Aminoalkylnitrils gemäß der vorliegenden Erfindung und
- Figur 2: das schematische Fließbild zur Herstellung eines Diamins aus einem entsprechenden Aminoalkylnitril gemäß der vorliegenden Erfindung.

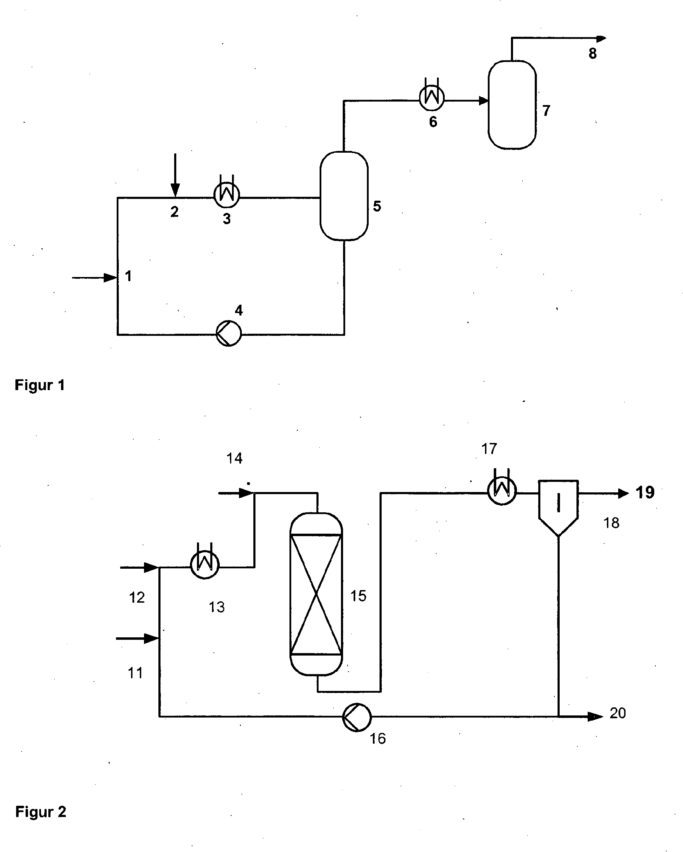

Figur 1 zeigt die Stufe der Umsetzung des Monoamins mit Alkenylnitril zum entsprechenden Aminoalkylnitril. Hierbei wird in einer Kreisfahrweise das Monoamin über Einlass 1 und das Alkenylnitril über Einlass 2 dem Kreisstrom zugeführt. Die eigentliche Umsetzung des Monoamins mit dem Alkenylnitril erfolgt im Reaktor 5 über eine Rohrleitung, die zwischen Einlass 2 und Reaktor 5 einen Wärmetauscher 3 vorsieht. Der sich in Reaktor 5 befindliche Reaktionsstrom wird teilweise mit Hilfe einer Pumpe 4 in den Kreisstrom zurückgeführt, wobei der nicht rückgeführte Teil des Reaktionsstromes über einen Wärmetauscher 6 in einen zweiten Reaktor 7 überführt wird. Hierdurch ist gewährleistet, dass der Reaktionsstrom in den Reaktoren 5 und 7 eine unterschiedliche Temperatur aufweisen kann. Der Reaktionsstrom, der Aminoalkylnitril enthält, verlässt über Auslass 8 den Reaktor 7.

Figur 2 zeigt die Reduktionsstufe mit Hilfe von Wasserstoff. Das aus Auslass 8 stammende Aminoalkylnitril wird über Einlass 14 in einen Festbettreaktor 15 mit Hilfe einer Rohrleitung überführt. Hierbei dient das Katalysator-Festbett zur Reduktion der Nitrilgruppe mit Hilfe von Wasserstoff. Dieser wird über Einlass 12 zugeführt. Über Einlass 11 wird Ammoniak zugeführt, wobei der Reaktionsstrom derart geleitet wird, dass nach Zugabe von Wasserstoff und Ammoniak ein Wärmetauscher 13 zu deren Erwärmung durchlaufen wird, bevor das Aminoalkylnitril über Einlass 14 in den Reaktor 15 gelangt. Dies hat insbesondere den Vorteil, dass das Aminoalkylnitril möglichst rasch im Reaktor 15 umgesetzt wird und dadurch nur eine sehr geringe Verweilzeit bis zum Kontaktieren mit dem Hydrierkatalysator hat. Hierdurch kann erreicht werden, dass weniger Nebenprodukt (Bis-Diamin) gebildet wird. Der Festbettreaktor 15 wird von oben nach unten durchströmt (Rieselfahrweise) und gelangt über den Wärmetauscher 17 zum Abscheider 18, bei dem das Rohprodukt von Wasserstoff, Monoamin und Ammoniak abgetrennt und über den Ausgang 20 ausgeführt werden kann. Zudem kann das Rohprodukt zumindest teilweise mit Hilfe der Pumpe 16 dem Reaktionsstrom wieder zugeführt werden. Bei zumindest teilweiser Rückführung entsteht auch bei der Hydrierung ein Kreisprozess, was bevorzugt ist. Das anfallende Gemisch aus Wasserstoff, Monoamin und Ammoniak kann über Ausgang 19 ausgeführt werden.

### Beispiele

### Beispiel 1

Die Anlagerung von DMA an ACN wird in einer kontinuierlich betriebenen Rührkesselkaskade, bestehend aus drei Rührkesseln, durchgeführt. Das Volumenverhältnis der drei Rührkessel beträgt 1:1,5:2,5. Die Umsetzung wird bei einem Druck von 5 bar durchgeführt.

Die Temperatur in den ersten zwei Rührkesseln beträgt 60°C, im dritten Kessel 30°C. Die Anlagerung wird bei einer Belastung, gerechnet als Zulauf Acrylnitril pro Zeiteinheit, bezogen auf das Volumen des ersten Rührbehälters, von 0,54 kg/L/h durchgeführt. Das molare Einsatzstoffverhältnis von DMA zu ACN beträgt 0,98. Der Acrylnitril-Restgehalt beträgt 3 Gew.-% (bezogen auf den gesamten Reaktionsaustrag). Die mittlere Verweilzeit des Reaktionsgemisches in den Behältern der Anlagerungsstufe beträgt ca. 4 Stunden.

Die Reaktionsausträge der Anlagerungsstufe werden in einer zweiten Reaktionsstufe in einem kontinuierlich betriebenen Reaktor an einem Cobalt-haltigen Festbettkatalysator hydriert. Die Katalysatorbelastung beträgt 1,0 kg(DMAPN-Rohaustrag)/kg(Katalysator)/h. Die Hydrierung wird bei einem Absolutdruck von 180 bar durchgeführt. Die Reaktorausgangstemperatur beträgt 120 °C. Das Massenverhältnis von Wälzstrom zu DMAPN-Zulauf beträgt 2,5 kg/kg. Der Anteil an Ammoniak im Reaktionszulauf beträgt 0,8 kg (Ammoniak)/kg (DMAPN), das molare Verhältnis von Ammoniak zu Nitril beträgt 5.

Nach einer Laufzeit von insgesamt 1300 h wird in der Hydrierung ein merklicher Nitrildurchbruch ermittelt (> 0,1 % DMAPN im Austrag).

### Beispiel 2

Die Anlagerung von DMA an ACN wird in einer kontinuierlich betriebenen Rührkesselkaskade, bestehend aus sieben Rührkesseln, durchgeführt. Das Volumenverhältnis der sieben Rührkessel beträgt 1:1,5:2,5:3,4:3,4:3,4:3,4. Die Umsetzung wird bei einem Druck von 5 bar durchgeführt.

Die Temperatur in den ersten zwei Rührkesseln beträgt 60°C, im dritten bis siebten Kessel 40°C. Die Anlagerung wird bei einer Belastung, gerechnet als Zulauf Acrylnitril pro Zeiteinheit, bezogen auf das Volumen des ersten Rührbehälters, von 0,54 kg/L/h durchgeführt. Das molare Einsatzstoffverhältnis von DMA zu ACN beträgt 0,98. Der Acrylnitril-Restgehalt beträgt ca. 2 Gew.-% (bezogen auf den gesamten Reaktionsaustrag). Die mittlere Verweilzeit des Reaktionsgemisches in den Behältern der Anlagerungsstufe beträgt ca. 16 Stunden.

Nach einer Laufzeit von 500 h wird für ca. 24 h eine verringerte DMA-Menge in den Anlagerungsreaktor gegeben, was zu einem Anstieg des Rest-Acrylnitrilgehaltes im Austrag auf 5 bis 30 Gew.% führt.

Die Reaktionsausträge der Anlagerungsstufe werden in einer zweiten Reaktionsstufe in einem kontinuierlich betriebenen Reaktor an einem Cobalt-haltigen Festbett-Katalysator hydriert. Die Katalysatorbelastung beträgt 1,0 kg(DMAPN-Rohaustrag)/kg(Katalysator)/h. Die Hydrierung wird bei einem Absolutdruck von 180 bar durchgeführt. Die Reaktorausgangstemperatur beträgt 120°C. Das Massenverhältnis von Wälzstrom zu DMAPN-Zulauf beträgt 2,5 kg/kg. Der Anteil an Ammoniak im Reaktionszulauf beträgt 0,8 kg (Ammoniak)/kg (DMAPN), das molare Verhältnis von Ammoniak zu Nitril beträgt 5.

Innerhalb der ersten 500 h Laufzeit wird kein Nitrildurchbruch im Austrag der Hydrierstufe nachgewiesen.

Nach einer Laufzeit von insgesamt 530 h wird in der Hydrierung ein merklicher Nitrildurchbruch ermittelt (> 0,2 % DMAPN im Austrag).

### Beispiel 3

Die Anlagerung von DMA an ACN wird in einer kontinuierlich betriebenen Rührkesselkaskade, bestehend aus drei Rührkesseln, durchgeführt. Das Volumenverhältnis der drei Rührkessel beträgt 1:1,5:2,5. Die Umsetzung wird bei einem Druck von 5 bar durchgeführt.

Die Temperatur in den ersten zwei Rührkesseln beträgt 60 bis 80°C, im dritten Kessel 30 bis 45°C. Die Anlagerung wird bei einer Belastung, gerechnet als Zulauf Acrylnitril pro Zeiteinheit, bezogen auf das Volumen des ersten Rührbehälters, von 0,44 bis 0,54 kg/L/h durchgeführt. Das molare Einsatzstoffverhältnis von DMA zu ACN beträgt 1,08 bis 1,18. Der Acrylnitril-Restgehalt beträgt weniger als 0,2 Gew.-% (bezogen auf den gesamten Reaktionsaustrag). Die mittlere Verweilzeit des Reaktionsgemisches in den Behältern der Anlagerungsstufe beträgt ca. 4 Stunden.

Die Reaktionsausträge der Anlagerungsstufe werden in einer zweiten Reaktionsstufe in einem kontinuierlich betriebenen Reaktor an einem Cobalt-haltigen Festbettkatalysator hydriert. Die Katalysatorbelastung beträgt 1,0 kg(DMAPN-Rohaustrag)/kg(Katalysator)/h. Die Hydrierung wird bei einem Absolutdruck von 180 bar durchgeführt. Die Reaktorausgangstemperatur beträgt 120°C. Das Massenverhältnis von Wälzstrom zu DMAPN-Zulauf beträgt 2,5 kg/kg. Der Anteil an Ammoniak im Reaktionszulauf beträgt 0,8 kg (Ammoniak)/kg (DMAPN), das molare Verhältnis von Ammoniak zu Nitril beträgt 5.

Innerhalb einer Laufzeit von 600 h wird in der Hydrierung kein Nitrildurchbruch ermittelt.

### Beispiel 4

Die Anlagerung von DMA an ACN wird in einer kontinuierlich betriebenen Anlage, bestehend aus einem umgepumpten Kreis mit zwei Behältern sowie einem laminar durchströmten Behälter und zwei weiteren Behältern, durchgeführt. Das Volumenverhältnis der Behälter des Umpumpkreises zum laminar durchströmten Behälter und den zwei weiteren Behälter beträgt 1:0,2:2:3.

Die Temperatur im Umpumpkreis beträgt 60°C, in den weiteren Behältern 30 bis 45°C. Die Anlagerung wird bei einer Belastung, gerechnet als Zulauf Acrylnitril pro Zeiteinheit, bezogen auf das Volumen des Umpumpkreises, von 0,75 kg/L/h durchgeführt. Das Verhältnis von in den ersten zwei Behältern umgepumpter Menge Roh-DMAPN zu Reaktionsaustrag beträgt 30 kg/kg.

Das molare Einsatzstoffverhältnis von DMA zu ACN beträgt 1,08. Der Acrylnitril-Restgehalt beträgt weniger als 0,1 Gew.-% (bezogen auf den gesamten Reaktionsaustrag) im Reaktionsstrom nach dem 5. Behälter. Die mittlere Verweilzeit des Reaktionsgemisches in den Behältern der Anlagerungsstufe beträgt ca. 4 Stunden. Die Umsetzung wird bei einem Druck von 4 bar durchgeführt. Das eingesetzte Acrylnitril besitzt einen Wassergehalt von ca. 0,4 %.

Die Reaktionsausträge der Anlagerungsstufe werden in einer zweiten Reaktionsstufe in einem kontinuierlich betriebenen Reaktor an einem Cobalt-haltigen Festbett-Katalysator hydriert. Die Katalysatorbelastung beträgt im Schnitt 1,0 kg (DMAPN-Rohaustrag)/kg(Katalysator)/h. Die Hydrierung wird bei einem Absolutdruck von 180 bar durchgeführt. Die Reaktorausgangstemperatur im Reaktor beträgt ca. 144°C. Das Massenverhältnis von Wälzstrom zu DMAPN-Zulauf beträgt 2,5 kg/kg. Der Anteil an Ammoniak im Reaktionszulauf beträgt 0,8 kg (Ammoniak)/kg (DMAPN), das molare Verhältnis von Ammoniak zu Nitril beträgt 5.

Innerhalb einer Laufzeit von 6 Monaten wird in der Hydrierung kein Nitrildurchbruch ermittelt, in dieser Zeit wurde eine Gesamtmenge von ca. 4500 kg (DMAPN)/kg(Katalysator) hydriert.

## Patentansprüche

1. Verfahren zur Herstellung eines Aminoalkylnitrils durch Umsetzung eines entsprechenden Monoamins mit einem entsprechenden Alkenylnitril in kontinuierlicher Fahrweise, die Schritte enthaltend
(a) Zuführen des entsprechenden Monoamins in einen kontinuierlich gefahrenen Reaktionsstrom;
(b) Zuführen des entsprechenden Alkenylnitrils in den Reaktionsstrom, wobei dieser bei Zugabe bereits das Aminoalkylnitril enthält;
(c) Umsetzung des Reaktionsstromes in einem ersten Reaktionsbereich; und
(d) zumindest teilweises Überführen des Reaktionsstromes in zumindest einen zweiten Reaktionsbereich zur weiteren Umsetzung.

2. Verfahren nach Anspruch 1, **dadurch gekennzeichnet, dass** es sich bei dem Aminoalkylnitril um 3-Dimethylaminopropionitril handelt.

3. Verfahren nach Anspruch 1 oder 2, **dadurch gekennzeichnet, dass** zumindest die Schritte (a) bis (c) in Kreisstromfahrweise durchgeführt werden.

4. Verfahren nach einem der Ansprüche 1 bis 3, **dadurch gekennzeichnet, dass** das Alkenylnitril und das Monoamin in flüssiger Form zugeführt werden.

5. Verfahren nach einem der Ansprüche 1 bis 4, **dadurch gekennzeichnet, dass** bei Zugabe des entsprechenden Alkenylnitrils der Reaktionsstrom einen Mindestüberschuss an entsprechendem Monoamin von 0,1 Mol-% in Bezug auf das Alkenylnitril aufweist.

6. Verfahren nach einem der Ansprüche 1 bis 5, **dadurch gekennzeichnet, dass** die Umsetzung in Schritt (c) derart erfolgt, dass der Verbrauch an Alkenylnitril im Bereich von 80 bis 98 % liegt.

7. Verfahren nach einem der Ansprüche 1 bis 6, **dadurch gekennzeichnet, dass** nach der Umsetzung in Schritt (d) der Restgehalt an Alkenylnitril im Reaktionsstrom unter 1 Gew.-% liegt.

8. Verfahren nach einem der Ansprüche 1 bis 7, **dadurch gekennzeichnet, dass** der Reaktionsstrom in erstem und zweitem Reaktionsbereich bei der Umsetzung unterschiedliche Temperaturen aufweist.
B05/1175PC

9. Verfahren nach einem der Ansprüche 1 bis 8, **dadurch gekennzeichnet, dass** der Reaktionsstrom im ersten Reaktionsbereich bei der Umsetzung eine höhere Temperatur als im zumindest zweiten Reaktionsbereich aufweist.

10. Verfahren nach einem der Ansprüche 1 bis 9, **dadurch gekennzeichnet, dass** die Temperatur des Reaktionsstromes im ersten Reaktionsbereich im Bereich von 20°C bis 110°C liegt.

11. Verfahren nach einem der Ansprüche 1 bis 10, **dadurch gekennzeichnet, dass** die Temperatur des Reaktionsstromes im zumindest zweiten Reaktionsbereich im Bereich von 0°C bis 100°C liegt.

12. Verfahren nach einem der Ansprüche 1 bis 11, **dadurch gekennzeichnet, dass** der Überdruck in dem ersten und zweiten Reaktionsbereich gleich oder verschieden ist und im Bereich von 1 bar bis 20 bar liegt.

13. Verfahren nach einem der Ansprüche 1 bis 12, **dadurch gekennzeichnet, dass** dem Reaktionsstrom vor der Umsetzung im ersten Reaktionsbereich Wasser zugeführt wird.

14. Verfahren nach einem der Ansprüche 1 bis 13, **dadurch gekennzeichnet, dass** das Monoamin in Form einer wässrigen Lösung dem Reaktionsstrom zugeführt wird.

15. Verfahren nach einem der Ansprüche 1 bis 14, **dadurch gekennzeichnet, dass** dem Reaktionsstrom nach der Umsetzung im zweiten Reaktionsbereich nicht umgesetztes. Monoamin in oder außerhalb des zweiten Reaktionsbereiches entzogen wird.

16. Verfahren nach einem der Ansprüche 1 bis 15 den weiteren Schritt enthaltend
(e) Ausführen des Reaktionsstromes nach der Umsetzung aus dem zweiten Reaktionsbereich.

17. Verfahren zur Herstellung eines Diamins aus einem entsprechenden Aminoalkylnitril, welches durch Umsetzung eines entsprechenden Monoamins mit einem entsprechenden Alkenylnitril in kontinuierlicher Fahrweise hergestellt wird, die Schritte enthaltend
(a) Zuführen des entsprechenden Monoamins in einen kontinuierlich gefahrenen Reaktionsstrom;
(b) Zuführen des entsprechenden Alkenylnitrils in den Reaktionsstrom, wobei dieser bei Zugabe bereits das Aminoalkylnitril enthält;
(c) Umsetzung des Reaktionsstromes in einem ersten Reaktionsbereich; und
(d) zumindest teilweises Überführen des Reaktionsstromes in zumindest einen zweiten Reaktionsbereich zur weiteren Umsetzung;
(e) Ausführen des Reaktionsstromes nach der Umsetzung aus dem zweiten Reaktionsbereich;
(f) Einleiten des in Schritt (e) ausgeleiteten Reaktionsstromes in einen Reduktionsbereich; und
(g) Reduktion des im Reaktionsstrom befindlichen Aminoalkylnitrils zum entsprechenden Diamin.

18. Verfahren nach Anspruch 17, **dadurch gekennzeichnet, dass** die Reduktion mit Hilfe von Wasserstoff erfolgt.

19. Verfahren nach Anspruch 17 oder 18, **dadurch gekennzeichnet, dass** vor Schritt (g) dem Reaktionsstrom Ammoniak zugeführt wird.

20. Verfahren nach einem der Ansprüche 17 bis 19, **dadurch gekennzeichnet, dass** die Schritte (f) und (g) in kontinuierlicher Fahrweise durchgeführt werden.

21. Verfahren nach Anspruch 20, **dadurch gekennzeichnet, dass** das Rohprodukt aus Schritt (g) von Anspruch 17 zumindest teilweise in einem Kreisstrom zurückgeführt wird.

22. Verfahren nach Anspruch 18, 19 und 21, **dadurch gekennzeichnet, dass** Wasserstoff und Ammoniak in Richtung des Kreisstroms vor der Einleitung des Reaktionsstromes in Schritt (f) von Anspruch 17 dem Kreisstrom zugeführt werden.

23. Verfahren nach Anspruch 22, **dadurch gekennzeichnet, dass** nach Zuführung des Wasserstoffs sowie des Ammoniaks und vor dem Einleiten in Schritt (f) von Anspruch 17 der Kreisstrom einen Wärmetauscher zu dessen Erwärmung durchläuft.

24. Verfahren nach einem der Ansprüche 17 bis 23, **dadurch gekennzeichnet, dass** die Reduktion an einem Festbett-Katalysator erfolgt.

25. Verfahren nach einem der Ansprüche 17 bis 24, **dadurch gekennzeichnet, dass** die maximale Temperatur im Reduktionsbereich während der Reduktion 150 °C beträgt.

26. Verfahren nach einem der Ansprüche 17 bis 25, **dadurch gekennzeichnet, dass** die Reduktion adiabatisch erfolgt.

27. Vorrichtung zur Herstellung eines Aminoalkylnitrils durch Umsetzung eines entsprechenden Monoamins mit einem entsprechenden Alkenylnitril in kontinuierlicher Fahrweise enthaltend
- einen ersten Reaktionsbereich enthaltend
- einen Einlass mit einer Zuleitung, wobei die Zuleitung
- einen ersten Einlass, der geeignet ist, der Zuleitung das Monoamin zuzuführen;
- einen zweiten Einlass, der geeignet ist das Alkenylnitril der Zuleitung zuzuführen und der zwischen erstem Einlass und Einlass des ersten Reaktionsbereiches liegt, aufweist;
- einen Auslass, der mit der Zuleitung verbunden ist; und
- eine Überführung zu einem zweiten Reaktionsbereich; und
- einen zweiten Reaktionsbereich,
wobei die Zuleitung das Aminoalkylnitril enthält.

28. Vorrichtung nach Anspruch 27, **dadurch gekennzeichnet, dass** der erste und zweite Reaktionsbereich unterschiedliche Reaktorbehälter darstellen.

29. Vorrichtung nach Anspruch 28, **dadurch gekennzeichnet, dass** die Überführung der unterschiedlichen Reaktorbehälter eine Rohrleitung darstellt.

30. Vorrichtung zur Herstellung eines Diamins aus einem entsprechenden Aminoalkylnitril enthaltend
- eine Vorrichtung nach einem der Ansprüche 27 bis 29; und
- einen Reduktionsbereich.

31. Vorrichtung nach Anspruch 30, **dadurch gekennzeichnet, dass** der Reduktionsbereich ein Festbettreaktor ist.

32. Verwendung einer Vorrichtung nach einem der Ansprüche 27 bis 29 zur Herstellung eines Aminoalkylnitrils durch Umsetzung eines entsprechenden Monoamins mit einem entsprechenden Alkenylnitril in kontinuierlicher Fahrweise.

33. Verwendung einer Vorrichtung nach Anspruch 30 oder 31 zur Herstellung eines Diamins aus einem entsprechenden Aminoalkylnitril.

## Claims

1. A process for preparing an aminoalkyl nitrile by reaction of a corresponding monoamine with a corresponding alkenyl nitrile in a continuous mode of operation, which comprises the steps
(a) introduction of the corresponding monoamine into a continuously conveyed reaction stream;
(b) introduction of the corresponding alkenyl nitrile into the reaction stream, with this already comprising the aminoalkyl nitrile on addition;
(c) reaction of the reaction stream in a first reaction region; and
(d) at least partial transfer of the reaction stream into at least one second reaction region for further reaction.

2. The process according to claim 1, wherein the aminoalkyl nitrile is 3-dimethylaminopropionitrile.

3. The process according to claim 1 or 2, wherein at least the steps (a) to (c) are carried out in the recycle mode.

4. The process according to any of claims 1 to 3, wherein the alkenyl nitrile and the monoamine are introduced in liquid form.

5. The process according to any of claims 1 to 4, wherein, on addition of the corresponding alkenyl nitrile, the reaction stream has a minimum excess of corresponding monoamine of 0.1 mol% based on the alkenyl nitrile.

6. The process according to any of claims 1 to 5, wherein the reaction in step (c) is carried out so that the consumption of alkenyl nitrile is in the range from 80 to 98%.

7. The process according to any of claims 1 to 6, wherein the residual content of alkenyl nitrile in the reaction stream after the reaction in step (d) is less than 1% by weight.

8. The process according to any of claims 1 to 7, wherein the reaction stream in the first and second reaction regions in the reaction have different temperatures.

9. The process according to any of claims 1 to 8, wherein the reaction stream in the first reaction region in the reaction has a higher temperature than in the at least second reaction region.

10. The process according to any of claims 1 to 9, wherein the temperature of the reaction stream in the first reaction region is in the range from 20°C to 110°C.

11. The process according to any of claims 1 to 10, wherein the temperature of the reaction stream in the at least second reaction region is in the range from 0 to 100°C.

12. The process according to any of claims 1 to 11, wherein the gauge pressure in the first and second reaction regions is identical or different and is in the range from 1 bar to 20 bar.

13. The process according to any of claims 1 to 12, wherein water is introduced into the reaction stream before the reaction in the first reaction region.

14. The process according to any of claims 1 to 13, wherein the monoamine is introduced in the form of an aqueous solution into the reaction stream.

15. The process according to any of claims 1 to 14, wherein unreacted monoamine is separated off from the reaction stream after the reaction in the second reaction region either in or outside the second reaction region.

16. The process according to any of claims 1 to 15 which comprises the further step
(e) discharge of the reaction stream from the second reaction region after the reaction.

17. A process for preparing a diamine from a corresponding aminoalkyl nitrile which is prepared by reaction of a corresponding monoamine with a corresponding alkenyl nitrile in a continuous mode of operation, which comprises the steps:
(a) introduction of the corresponding monoamine into a continuously conveyed reaction stream;
(b) introduction of the corresponding alkenyl nitrile into the reaction stream, with this already comprising the aminoalkyl nitrile on addition;
(c) reaction of the reaction stream in a first reaction region; and
(d) at least partial transfer of the reaction stream into at least one second reaction region for further reaction;
(e) discharge of the reaction stream from the second reaction region after the reaction;
(f) introduction of the reaction stream discharged in step (e) into a reduction region; and
(g) reduction of the aminoalkyl nitrile present in the reaction stream to the corresponding diamine.

18. The process according to claim 17, wherein the reduction is effected by means of hydrogen.

19. The process according to claim 17 or 18, wherein ammonia is introduced into the reaction stream before step (g).

20. The process according to any of claims 17 to 19, wherein steps (f) and (g) are carried out in a continuous mode of operation.

21. The process according to claim 20, wherein the crude product from step (g) of claim 17 is at least partly recirculated in a recycle stream.

22. The process according to any of claims 18, 19 and 21, wherein hydrogen and ammonia are introduced into the recycle stream before, in the direction of the recycle stream, introduction of the reaction stream into step (f) of claim 17.

23. The process according to claim 22, wherein the recycle stream goes through a heat exchanger to heat it after introduction of hydrogen and of ammonia and before introduction into step (f) of claim 17.

24. The process according to any of claims 17 to 23, wherein the reduction is carried out over a fixed-bed catalyst.

25. The process according to any of claims 17 to 24, wherein the maximum temperature in the reduction region during the reduction is 150°C.

26. The process according to any of claims 17 to 25, wherein the reduction is carried out adiabatically.

27. An apparatus for preparing an aminoalkyl nitrile by reaction of a corresponding monoamine with a corresponding alkenyl nitrile in a continuous mode of operation, which comprises
- a first reaction region comprising
- an inlet provided with a feed line, with the feed line having
- a first inlet which is suitable for introducing the monoamine into the feed line;
- a second inlet which is suitable for introducing the alkenyl nitrile into the feed line and is located between the first inlet and the inlet of the first reaction region;
- an outlet which is connected to the feed line; and
- a transfer facility to a second reaction region; and
- a second reaction region,
with the feed line comprising the aminoalkyl nitrile.

28. The apparatus according to claim 27, wherein the first and second reaction regions are different reactor vessels.

29. The apparatus according to claim 28, wherein the transfer facility between the various reactor vessels is a pipe.

30. An apparatus for preparing a diamine from a corresponding aminoalkyl nitrile, which comprises
- an apparatus according to any of claims 27 to 29; and
- a reduction region.

31. The apparatus according to claim 30, wherein the reduction region is a fixed-bed reactor.

32. The use of an apparatus according to any of claims 27 to 29 for preparing an aminoalkyl nitrile by reaction of a corresponding monoamine with a corresponding alkenyl nitrile in a continuous mode of operation.

33. The use of an apparatus according to claim 30 or 31 for preparing a diamine from a corresponding aminoalkyl nitrile.

## Revendications

1. Procédé pour la production d'un aminoalkylnitrile par mise en réaction d'une monoamine correspondante avec un alcénylnitrile correspondant, en mode opératoire en continu, comportant les étapes
(a) introduction de la monoamine correspondante dans un courant réactionnel conduit en continu ;
(b) introduction de l'alcénylnitrile correspondant dans le courant réactionnel, ce dernier comportant déjà l'aminoalkylnitrile lors de l'addition ;
(c) mise en réaction du courant réactionnel dans une première zone de réaction ; et
(d) transfert au moins partiel du courant réactionnel dans au moins une deuxième zone de réaction pour la réaction ultérieure.

2. Procédé selon la revendication 1, **caractérisé en ce que** l'aminoalkylnitrile consiste en 3-diméthyl-aminopropionitrile.

3. Procédé selon la revendication 1 ou 2, **caractérisé en ce qu'**au moins les étapes (a) à (c) sont effectuées en mode opératoire en circuit.

4. Procédé selon l'une quelconque des revendications 1 à 3, **caractérisé en ce que** l'alcénylnitrile et la monoamine sont introduits sous forme liquide.

5. Procédé selon l'une quelconque des revendications 1 à 4, **caractérisé en ce que** lors de l'addition de l'alcénylnitrile correspondant, le courant réactionnel présente un excès minimum de monoamine correspondante de 0,1 % en moles par rapport à l'alcénylnitrile.

6. Procédé selon l'une quelconque des revendications 1 à 5, **caractérisé en ce que** la réaction dans l'étape (c) s'effectue de manière que la consommation d'alcénylnitrile se situe dans la plage de 80 à 98 %.

7. Procédé selon l'une quelconque des revendications 1 à 6, **caractérisé en ce qu'**après la réaction dans l'étape (d) la teneur résiduelle en alcénylnitrile du courant réactionnel est inférieure à 1 % en poids.

8. Procédé selon l'une quelconque des revendications 1 à 7, **caractérisé en ce que** lors de la réaction le courant réactionnel présente des températures différentes dans la première zone de réaction et dans la deuxième.

9. Procédé selon l'une quelconque des revendications 1 à 8, **caractérisé en ce que** lors de la réaction le courant réactionnel présente dans la première zone de réaction une température plus élevée que dans ladite au moins deuxième zone de réaction.

10. Procédé selon l'une quelconque des revendications 1 à 9, **caractérisé en ce que** dans la première zone de réaction la température du courant réactionnel se situe dans la plage allant de 20 °C à 110 °C.

11. Procédé selon l'une quelconque des revendications 1 à 10, **caractérisé en ce que** dans ladite au moins deuxième zone de réaction la température du courant réactionnel se situe dans la plage allant de 0 °C à 100 °C.

12. Procédé selon l'une quelconque des revendications 1 à 11, **caractérisé en ce que** dans les première et deuxième zones de réaction la surpression est la même ou différente et se situe dans la plage allant de 1 bar à 20 bars.

13. Procédé selon l'une quelconque des revendications 1 à 12, **caractérisé en ce qu'**avant la réaction dans la première zone de réaction on introduit de l'eau dans le courant réactionnel.

14. Procédé selon l'une quelconque des revendications 1 à 13, **caractérisé en ce qu'**on introduit dans le courant réactionnel la monoamine sous forme d'une solution aqueuse.

15. Procédé selon l'une quelconque des revendications 1 à 14, **caractérisé en ce qu'**après la réaction dans la deuxième zone de réaction on extrait du courant réactionnel, dans ou en dehors de la deuxième zone de réaction, la monoamine n'ayant pas réagi.

16. Procédé selon l'une quelconque des revendications 1 à 15, comportant l'étape supplémentaire
(e) après la réaction sortie du courant réactionnel de la deuxième zone de réaction.

17. Procédé pour la production d'une diamine à partir d'un aminoalkylnitrile correspondant, qui est produit par mise en réaction d'une monoamine correspondante avec un alcénylnitrile correspondant en mode opératoire en continu, comportant les étapes
(a) introduction de la monoamine correspondante dans un courant réactionnel conduit en continu ;
(b) introduction de l'alcénylnitrile correspondant dans le courant réactionnel, ce dernier comportant déjà l'aminoalkylnitrile lors de l'addition ;
(c) mise en réaction du courant réactionnel dans une première zone de réaction ; et
(d) transfert au moins partiel du courant réactionnel dans au moins une deuxième zone de réaction pour la réaction ultérieure ;
(e) après la réaction sortie du courant réactionnel de la deuxième zone de réaction ;
(f) introduction du courant réactionnel, sorti dans l'étape (e), dans une zone de réduction ; et
(g) réduction de l'aminoalkylnitrile, se trouvant dans le courant réactionnel, en la diamine correspondante.

18. Procédé selon la revendication 17, **caractérisé en ce que** la réduction s'effectue à l'aide d'hydrogène.

19. Procédé selon la revendication 17 ou 18, **caractérisé en ce qu'**avant l'étape (g) on ajoute de l'ammoniac au courant réactionnel.

20. Procédé selon l'une quelconque des revendications 17 à 19, **caractérisé en ce qu'**on effectue les étapes (f) et (g) en mode opératoire en continu.

21. Procédé selon la revendication 20, **caractérisé en ce que** le produit brut provenant de l'étape (g) de la revendication 17 est au moins en partie recyclé en un courant en circuit.

22. Procédé selon l'une quelconque des revendications 18, 19 et 21, **caractérisé en ce qu'**avant l'introduction du courant réactionnel dans l'étape (f) de la revendication 17 on envoie au courant en circuit de l'hydrogène et de l'ammoniac dans le sens du courant en circuit.

23. Procédé selon la revendication 22, **caractérisé en ce qu'**après introduction de l'hydrogène ainsi que de l'ammoniac et avant l'introduction dans l'étape (f) de la revendication 17 le courant en circuit traverse un échangeur thermique afin d'y être chauffé.

24. Procédé selon l'une quelconque des revendications 17 à 23, **caractérisé en ce que** la réduction s'effectue sur un catalyseur en lit fixe.

25. Procédé selon l'une quelconque des revendications 17 à 24, **caractérisé en ce que** la température maximale dans la zone de réduction pendant la réduction est de 150 °C.

26. Procédé selon l'une quelconque des revendications 17 à 25, **caractérisé en ce que** la réduction s'effectue en mode adiabatique.

27. Dispositif pour la production d'un aminoalkyl-nitrile par mise en réaction d'une monoamine correspondante avec un alcénylnitrile correspondant, en mode opératoire continu, comportant
- une première zone de réaction comportant
- une entrée avec un conduit d'alimentation, le conduit d'alimentation comportant
- une première entrée qui est appropriée pour l'introduction de la monoamine dans le conduit d'alimentation ;
- une deuxième entrée qui est appropriée pour l'introduction de l'alcénylnitrile dans le conduit d'alimentation et qui se trouve entre la première entrée et l'entrée de la première zone de réaction ;
- une sortie qui est reliée au conduit d'alimentation ; et
- un conduit de transfert menant à une deuxième zone de réaction ; et
- une deuxième zone de réaction,
le conduit d'alimentation contenant l'aminoalkyl-nitrile.

28. Dispositif selon la revendication 27, **caractérisé en ce que** les première et deuxième zones de réaction représentent des récipients de réaction différents.

29. Dispositif selon la revendication 28, **caractérisé en ce que** le conduit de transfert des différents récipients de réaction représente un conduit tubulaire.

30. Dispositif pour la production d'une diamine à partir d'un aminoalkylnitrile correspondant, comportant
- un dispositif selon l'une quelconque des revendications 27 à 29 ; et
- une zone de réduction.

31. Dispositif selon la revendication 30, **caractérisé en ce que** la zone de réduction est un réacteur à lit fixe.

32. Utilisation d'un dispositif selon l'une quelconque des revendications 27 à 29, pour la production d'un aminoalkylnitrile par mise en réaction d'une monoamine correspondante avec un alcénylnitrile correspondant, en mode opératoire en continu.

33. Utilisation d'un dispositif selon la revendication 30 ou 31, pour la production d'une diamine à partir d'un aminoalkylnitrile correspondant.
